(19) Europäisches Patentamt
European Patent Office
Office européen des brevets

(11) **EP 1 820 851 B1**

(12) **FASCICULE DE BREVET EUROPEEN**

(45) Date de publication et mention
de la délivrance du brevet:
**07.03.2012 Bulletin 2012/10**

(51) Int Cl.:
*C12N 1/20* (2006.01)     *C12N 15/01* (2006.01)
*A23C 9/123* (2006.01)     *A61K 35/74* (2006.01)

(21) Numéro de dépôt: **06290286.1**

(22) Date de dépôt: **20.02.2006**

(54) **Souches de Lactobacillus helveticus ne fermentant pas le lactose**

Laktose nicht fermentierende Lactobacillus helveticus

Non-lactose fermenting Lactobacillus helveticus

(84) Etats contractants désignés:
**AT BE BG CH CY CZ DE DK EE ES FI FR GB GR HU IE IS IT LI LT LU LV MC NL PL PT RO SE SI SK TR**

(43) Date de publication de la demande:
**22.08.2007 Bulletin 2007/34**

(73) Titulaire: **Compagnie Gervais Danone
75009 Paris (FR)**

(72) Inventeurs:
• **Garault, Peggy**
**91310 Monthery (FR)**
• **Druesne, Anne**
**91440 Bures sur Yvette (FR)**
• **Faurie, Jean-Michel**
**78350 Jouy en Josas (FR)**
• **Queguiner, Claire**
**92260 Fontenay Aux Roses (FR)**
• **Saint Denis, Thierry**
**94300 Vincennes (FR)**

(74) Mandataire: **Boulinguiez, Didier
Cabinet Plasseraud
52, rue de la Victoire
75440 Paris Cedex 09 (FR)**

(56) Documents cités:
**EP-A- 0 652 285     EP-A- 0 965 643
EP-A- 1 016 709     WO-A-00/15042
WO-A-01/88150     US-A- 4 452 895
US-A- 4 599 313**

• ZWAHLEN MARIE-CAMILLE ET AL: "ISL2, a new mobile genetic element in Lactobacillus helveticus" MOLECULAR AND GENERAL GENETICS, vol. 245, no. 3, 1994, pages 334-338, XP008066570 ISSN: 0026-8925
• PRESTINI P ET AL: "Correlation between plasmid DNA, lactose fermentation and proteolytic activity in Lactobacillus helveticus and Lactobacillus bulgaricus." ANNALI DELLA FACOLTA DI AGRARIA, UNIVERSITA CATTOLICA DEL SACRO CUORE, vol. 23, no. 1, 1983, pages 71-84, XP008066509
• SODA EL M ET AL: "ADJUNCT CULTURES: RECENT DEVELOPMENTS AND POTENTIAL SIGNIFICANCE TOTHE CHEESE INDUSTRY" JOURNAL OF DAIRY SCIENCE, AMERICAN DAIRY SCIENCE ASSOCIATION, SAVOY, IL, US, vol. 83, no. 4, avril 2000 (2000-04), pages 609-619, XP000924052 ISSN: 0022-0302
• FORTINA M GRAZIA ET AL: "Mapping of three plasmids from Lactobacillus helveticus ATCC 15009" LETTERS IN APPLIED MICROBIOLOGY, vol. 17, no. 6, 1993, pages 303-306, XP008066515 ISSN: 0266-8254

**EP 1 820 851 B1**

**Description**

La présente invention concerne de nouvelles souches de *Lactobacillus helveticus,* ainsi que leurs applications dans le domaine agro-alimentaire. Plus particulièrement, la présente invention propose des souches de *Lactobacillus helveticus* possédant un phénotype lactose moins. La présente invention concerne également un procédé d'obtention de telles souches de *Lactobacillus helveticus.*

L'hypertension touche une part importante de la population. L'utilisation du peptide VPP (Val Pro Pro) et de peptides contenant la séquence VPP comme agents capables de réduire la pression artérielle par inhibition de l'enzyme de conversion de l'angiotensine (ACE) a été décrite dans EP 0 583 074. Un effet similaire du tripeptide IPP (Ile Pro Pro) a été également décrit dans la littérature. Ces peptides inhibent l'ACE en bloquant son site actif et l'empêchent ainsi de rendre active l'angiotensine.

Il est connu que les deux séquences VPP et IPP sont présentes dans la caséine Béta bovine et que l'hydrolyse adéquate de cette caséine (ou plus généralement du lait qui la contient) permet d'obtenir lesdits tripeptides. De nombreuses études chez l'animal et chez l'homme ont montré que l'ingestion journalière de quelques milligrammes de ces tripeptides permet de réduire efficacement la pression artérielle, en particulier chez les sujets hypertendus, réduisant d'autant le risque d'accident cardio-vasculaire.

Des peptides issus d'un lait fermenté par des *Lactobacillus helveticus* ont montré in vivo leur effet inhibiteur de l'ACE (ACEI) et ces peptides ont pu être retrouvés au niveau de l'aorte des rats ayant participé à l'étude (Masuda O. et al., 1996. J. Nutr. 126, 3063-8). D'autres études ont aussi montré plus spécifiquement une diminution de la pression artérielle chez des rats hypertendus suite à l'ingestion de peptides issus d'un lait fermenté par des *L. helveticus* (Yamamoto N. et al., 1994. Biosci. Biotech. Biochem. 58, 776-8).

Chez l'Homme, le même lait fermenté a aussi permis de diminuer la pression artérielle systolique (Hata . et al. ; 1996. Am. J. Clin. Nutr., 64, 767-71). Une étude plus récente confirme que les peptides présents dans le produit AMEAL S ® commercialisé par la société CALPIS réduisent de manière significative la pression artérielle chez des sujets ayant une pression artérielle supérieure à la normale (Mizuno et al., 2005. British Journal of Nutrition ; vol 94, issue 1, 84-91).

Dans une autre étude récente (Jauhiainen et al. ; 2005. Am. J. of Hypertension, 18 : 1600-1605) est émis l'hypothèse selon laquelle le mécanisme d'action ci-dessus cité (inhibition de l'ACE) pour expliquer l'effet antihypertenseur d'un lait fermenté par un *L. helveticus* pourrait cependant ne pas être le mécanisme d'action déterminant chez l'homme.

Une grande majorité des *Lactobacillus helveticus* est capable de produire les tripeptides IPP et VPP par fermentation de lait, mais les quantités produites peuvent être variables. EP 1 016 709 décrit un moyen de produire les tripeptides VPP et IPP par fermentation de lait à l'aide de souches de bactéries lactiques spécifiques appartenant à l'espèce *Lactobacillus helveticus.* Toutefois, le produit ainsi fabriqué (lait fermenté) contient une très grande quantité d'acide lactique, et est donc caractérisé par une acidité inacceptable du point de vue sensoriel et gustatif. En outre se pose le problème de la post-acidification: même si la fermentation est arrêtée (classiquement par refroidissement) à des pH organoleptiquement acceptables (pH > 4), l'acidification se poursuit à froid du fait des propriétés intrinsèques des *Lactobacillus helveticus.* Ceci conduit à une chute du pH du produit, qui devient tout aussi inacceptable à consommer.

Afin d'éviter la post-acidification, il est possible d'arrêter la fermentation à un pH acceptable en tuant immédiatement la souche par traitement thermique (pasteurisation, thermisation) : la post-acidification est alors nulle (l'acidité du produit n'évolue plus pendant sa conservation). Malheureusement, la quantité de tripeptides est réduite du fait de l'arrêt précoce de la fermentation. Les produits ainsi obtenus n'ont alors que des teneurs faibles en IPP et VPP. Il est également possible de fermenter le lait par la même souche jusqu'à obtenir une concentration maximale en tripeptides. La grande quantité d'acide lactique générée en parallèle doit alors être retirée par un procédé multi étapes complexe et coûteux. En outre, le procédé n'est pas « naturel », car il fait intervenir des étapes de séparation. Un tel procédé n'est ainsi pas adapté à l'échelle industrielle.

La présente invention propose des solutions aux inconvénients de l'état de la technique. En particulier, la présente invention propose des souches de *Lactobacillus helveticus* permettant la préparation de produits alimentaires, notamment des produits laitiers fermentés, possédant de nombreuses propriétés avantageuses:

-    Les produits laitiers selon l'invention ont une acidité parfaitement contrôlée, non seulement en fin de fermentation, mais également au stockage. En particulier, ils ne sont pas sujets au phénomène de post-acidification. Ainsi, les produits laitiers selon l'invention sont organoleptiquement très satisfaisants.
-    Les produits laitiers selon l'invention sont directement consommables comme probiotiques, c'est-à-dire qu'ils contiennent une quantité significative de bactéries vivantes, sans aucun impact sur leurs qualités gustatives, et notamment, sans aucun impact sur leur acidité.
-    La préparation des produits selon l'invention ne nécessite aucune étape de thermisation, ni aucune étape complexe de purification. Les procédés selon l'invention peuvent ainsi être mis en oeuvre de manière simple, dans des installations de laiterie classique, sans modification importante de l'équipement. Par ailleurs, les procédés selon l'invention sont entièrement naturels.

- Les produits alimentaires, notamment laitiers, selon l'invention peuvent contenir de très fortes teneurs en tripeptides VPP et IPP, et ceci, avec des ratios élevés de (IPP+VPP) / (acide lactique). Ces résultats peuvent être obtenus sans étape de purification ou de concentration préalable.
- Les produits alimentaires, notamment laitiers, selon l'invention contiennent avantageusement une biomasse importante (i.e une concentration importante de micro-organismes vivants).

Par souche, on entend, au sens de la présente invention, toute culture, généralement pure, d'un micro-organisme, obtenue à partir d'une seule cellule ou d'une colonie isolée.

Par souche à « phénotype lactose moins », on entend toute souche n'ayant pas la capacité à transformer le lactose en acide lactique.

Par souche à « phénotype fructose moins », on entend toute souche n'ayant pas la capacité à métaboliser le fructose.

Par milieu laitier, on entend tout milieu contenant des protéines de lait, par exemple un lait bovin standardisé à 4% en protéines avec de la poudre de lait bovin (écrémé ou non) ou avec du lait concentré.

Par produit laitier, au sens de la présente invention, on entend, en plus du lait, tout produit dérivé du lait, tel la crème, la crème glacée, le beurre, le fromage, le yogourt, le lait fermenté; les produits secondaires, comme le lactosérum, la caséine ainsi que divers aliments préparés contenant comme ingrédient principal du lait ou des constituants du lait. Parmi les produits laitiers, les produits laitiers fermentés comprennent entre autres les yaourts, les laits fermentés, les fromages blancs, les kéfirs, les fromages, les produits laitiers probiotiques et plus généralement tout produit laitier qui a subi au moins une étape de fermentation. Ledit lait est généralement du lait de vache, mais peut également être du lait d'autres mammifères, comme la chèvre, la brebis, la jument, la chamelle, la bufflonne.

Par produit alimentaire, au sens de la présente invention, on entend tout produit destiné à la nutrition humaine ou animale. En particulier, les produits alimentaires comprennent des produits destinés à l'alimentation des nourrissons, des enfants, des adolescents, et des adultes. Les produits alimentaires selon l'invention peuvent contenir en tout ou partie, au moins un produit laitier fermenté selon l'invention. Les produits alimentaires selon l'invention peuvent également contenir d'autres ingrédients habituellement utilisés dans l'industrie agroalimentaire, tels que des additifs, des conservateurs, des fruits ou extraits de fruits, des arômes, des colorants, des agents de texture, des céréales, des morceaux de chocolat, etc.

Par ferment, on entend, au sens de la présente invention, toute composition contenant au moins une souche vivante de micro-organisme susceptible de fermenter un milieu donné. Parmi les ferments, les ferments lactiques sont des compositions contenant au moins une souche vivante de micro-organisme susceptible de fermenter un milieu laitier.

Selon un mode de réalisation, la présente invention concerne une souche de *Lactobacillus helveticus* n'ayant pas la capacité à transformer le lactose en acide lactique obtenue à partir des souches I-3431, I-3434 et I-3435 déposées à la CNCM le 25/05/05,
et capable, par fermentation à une température entre 30 et 45°C, plus préférentiellement entre 32 et 43°C et encore plus préférentiellement à 37°C d'un milieu laitier contenant une quantité de glucose supérieure à 3% (p/p) et avec une teneur totale en protéines supérieure ou égale à 2% (p/p), préférentiellement entre 2 et 10% (p/p), plus préférentiellement entre 2,5 et 6% et encore plus préférentiellement égale à 4%,
de produire les tripeptides de séquence IPP et/ou VPP en une quantité d'au moins 25 mg, préférentiellement au moins 50 mg, plus préférentiellement au moins 75 mg de VPPeq par kg de produit fermenté. La présente invention concerne également une souche de *Lactobacillus helveticus* n'ayant pas la capacité à transformer le lactose en acide lactique, et possédant au moins une mutation dans l'opéron lactose.

Ainsi, les souches *Lactobacillus helveticus* selon l'invention n'ont pas la capacité de dégrader le lactose (pas de transformation du lactose en acide lactique). En effet, les souches selon l'invention ont un phénotype lactose moins. En revanche, elles sont capables de croître en présence de glucose comme source de carbone (fermentation du glucose en acide lactique), et de métaboliser certains composants du lait, notamment les protéines.

Ainsi, les souches selon l'invention permettent avantageusement une fermentation à pH entièrement contrôlé: le pH final après fermentation est parfaitement maîtrisé selon la quantité de glucose initialement contenue dans le milieu de fermentation. Ceci résulte du fait qu'une quantité donnée de glucose résulte de manière quasiment stoechiométrique en la même quantité d'acide lactique. Par ailleurs, l'emploi des souches selon l'invention évite tout phénomène de post-acidification. Ainsi, grâce aux souches selon l'invention, il est possible d'obtenir des produits alimentaires particulièrement satisfaisants sur le plan organoleptique.

De manière avantageuse, selon un mode de réalisation, les souches de *Lactobacillus helveticus* selon l'invention sont telles qu'un milieu laitier à 4,0% de protéines totales contenant 1,0 % (p/p) de glucose fermenté par lesdites souches de *Lactobacillus helveticus* pendant un temps maximal de fermentation de 30h, à une température entre 30 et 45°C, possède un pH supérieur ou égal à 4,0 ; préférentiellement supérieur ou égal à 4,1 ; préférentiellement supérieur ou égal 4,2 ; préférentiellement supérieur ou égal à 4,3 ; préférentiellement supérieur ou égal à 4,5.

Cet avantage est donc particulièrement marqué dans le cas de souches de *Lactobacillus helveticus* hyperproductrices en tripeptides IPP et/ou VPP : il n'est absolument pas requis de choisir un compromis entre

- une forte production en tripeptides lors de la fermentation (nécessitant un temps de fermentation aussi long que possible), et
- des propriétés organoleptiquement acceptables (nécessitant une acidification limitée, et donc, non seulement un temps de fermentation aussi court que possible, afin de limiter la production d'acide lactique par fermentation, mais également une thermisation/pasteurisation afin d'éviter le phénomène de post-acidifcation).

En effet, avantageusement selon la présente invention, la production de tripeptides VPP et/ou IPP, et la production d'acide lactique sont partiellement découplées : la production d'acide lactique n'est que fonction de la quantité de glucose initialement présente dans le milieu de fermentation, et non plus fonction du temps de fermentation.

Un moyen d'exprimer la concentration en tripeptides de manière simple est de l'exprimer en concentration équivalent VPP [VPPeq].

On l'exprime en mg/kg : [VPPeq] = [VPP] + (9/5 x [IPP])

Ainsi, selon un mode de réalisation, les souches de *Lactobacillus helveticus* selon l'invention sont avantageusement capables, par fermentation de produire les tripeptides de séquence IPP et/ou VPP en une quantité d'au moins 25 mg, préférentiellement au moins 30 mg, préférentiellement au moins 35 mg, préférentiellement au moins 40 mg, préférentiellement au moins 45 mg, préférentiellement au moins 50 mg, préférentiellement au moins 55 mg, préférentiellement au moins 60 mg, préférentiellement au moins 65 mg, préférentiellement au moins 70 mg, préférentiellement au moins 75 mg, plus préférentiellement au moins 80 mg de VPPcq par kg de produit fermenté. De telles quantités de VPP et/ou IPP sont obtenues par fermentation par une souche selon l'invention à une température entre 30 et 45°C, préférentiellement entre 31 et 44°C, préférentiellement entre 32 et 43°C, préférentiellement entre 33 et 42°C, préférentiellement entre 34 et 41°C, préférentiellement entre 35 et 40°C, et plus préférentiellement à 37°C, d'un milieu laitier contenant une quantité de glucose supérieure à 3% (p/p) et avec une teneur totale en protéines supérieure ou égale à 2% (p/p), préférentiellement entre 2 et 10% (p/p), plus préférentiellement entre 2,5 et 6% (p/p) et encore plus préférentiellement égale à 4% (p/p),

Selon un mode de réalisation, les souches de *Lactobacillus helveticus* selon l'invention possèdent en outre un phénotype fructose moins. La combinaison des phénotypes lactose moins et fructose moins est particulièrement avantageuse. En effet, le fructose a un fort pouvoir sucrant, et est un ingrédient utile dans les produits alimentaires. Ainsi, si le fructose ne peut être dégradé par la souche de *Lactobacillus helveticus,* le produit alimentaire conserve d'excellentes propriétés organoleptiques. De plus, une souche à la fois lactose moins et fructose moins ne pourra pousser que dans un milieu contenant du glucose. Ainsi, le pH final sera avantageusement mieux contrôle dans le cas où le produit alimentaire contient une préparation de fruit(s), notamment des morceaux et/ou du jus de fruit(s).

Selon un mode de réalisation, la souche selon l'invention est choisie parmi:

| I-3504 | déposée à la CNCM le 14/10/05 ; |
| I-3505 | déposée à la CNCM le 14/10/05 ; |
| I-3508 | déposée à la CNCM le 14/10/05. |

Selon un autre mode de réalisation, la présente invention concerne l'utilisation d'une souche de *Lactobucillus selon* l'invention, pour la préparation d'un produit alimentaire ou pharmaceutique, notamment d'un produit laitier fermenté.

De manière avantageuse, selon un aspect de l'invention, ledit produit alimentaire ou pharmaceutique possède des propriétés anti-hypertensives.

La présente invention concerne également un produit alimentaire, notamment produit laitier fermenté, comprenant au moins une souche de *Lactobacillus helveticus* selon l'invention.

De manière avantageuse, selon un mode de réalisation, ledit produit alimentaire contient au moins $10^6$, préférentiellement au moins $10^7$, préférentiellement au moins $10^8$ UFC/mL de bactéries *Lacrobacillus helveticus* vivantes. Ainsi, le produit alimentaire selon l'invention contient une importante biomasse.

Selon un mode de réalisation, ledit produit alimentaire contient au moins 25 mg, préférentiellement au moins 30 mg, préférentiellement au moins 35 mg, préférentiellement au moins 40 mg, préférentiellement au moins 45 mg, préférentiellement au moins 50 mg, préférentiellement au moins 55 mg, préférentiellement au moins 60 mg, préférentiellement au moins 65 mg, préférentiellement au moins 70 mg, préférentiellement au moins 75 mg, plus préférentiellement au moins 80 mg de VPPeq par kg de produit alimentaire.

Selon un mode de réalisation, le produit alimentaire selon l'invention comprend en outre des préparations de fruit(s), notamment des morceaux et/ou du jus de fruit(s). Selon un mode de réalisation, le produit alimentaire selon l'invention possède un pH supérieur ou égal à 3,85 ; préférentiellement supérieur ou égal à 3.90 ; préférentiellement supérieur ou égal à 3,95 ; préférentiellement supérieure ou égal à 4,00 ; préférentiellement supérieur ou égal à 4,05 ; préférentiellement supérieur ou égal à 4,10 ; préférentiellement supérieur ou égal à 4,15 ; plus préférentiellement supérieur ou égal à 4,20.

Avantageusement selon un mode de réalisation, le produit alimentaire selon l'invention possède des propriétés anti-hypertensives.

Selon un autre aspect, la présente invention concerne un procédé de préparation d'un produit alimentaire.

Selon un mode de réalisation, ledit procédé comprend les étapes suivantes :

■ sélectionner une matière première contenant des protéines de lait, notamment des protéines contenant les séquences peptidiques IPP et/ou VPP. Ce peut par exemple être du lait contenant 2-10% (p/p) de protéines totales.
■ sélectionner au moins une souche *Lactobacillus helveticus* selon l'invention,
■ ensemencer ladite matière première avec ladite souche,
■ fermenter ladite matière première en présence de 1-3% (p/p) de glucose pendant 12-30 heures à 30-45°C, et en présence de ladite souche.

Selon un mode de réalisation, le procédé selon l'invention est avantageusement dépourvu do toute étape de thermisation après fermentation. Ceci permet de conserver des bactéries vivantes dans le produit alimentaire (probiotique).

La présente invention fournit également un procédé d'obtention de telles souches de *Lactobacillus helveticus.*

Selon un mode de réalisation, ledit procède d'obtention de souches *Lactobacillus helveticus* possédant un phénotype lactose moins, comprenant les étapes suivantes :

■ fournir au moins une souche de *Lactobacillus helveticus* choisie parmi les souches I-3431, I-3434 et I-3435 déposées à la CNCM le 25/05/05 ;
■ mettre en contact ladite au moins une souche avec une quantité efficace de streptozotocine en présence de lactose ;
■ isoler la ou les colonies de phénotype lactose moins.

La population de cellules de *Lactobacillus helveticus* capable de survivre en présence de streptozotocine est en effet avantageusement extrêmement enrichie en cellules lactose moins.

Selon un mode de réalisation, une étape de test colorimétrique sur un milieu contenant du Xgal et/ou du Sgal et/ou tout autre composé lié par une liaison β-galactosidique à du galactose et qui devient repérable suite à la rupture de cette liaison par le microorganisme, est ajoutée au-dit procédé d'obtention de souches selon l'invention. L'IPTG peut être également ajouté pour réaliser ce test puisque ce composé agira comme un inducteur du transporteur du lactose ou comme un inducteur de l'utilisation du lactose par le microorganisme.

A titre d'exemple, ledit procédé peut être conduit de la manière suivante:

- 1er repiquage des souches *Lactobacillus helveticus* de départ (souches mères), sur bouillon MRS (Man Rogosa Sharp) neutre, la nuit à 35-40°C ;
- 2ème repiquage dans du MRS neutre.
- Incubation jusqu'à obtention d'une biomasse suffisante, par exemple 10-30h à 35-40°C.
- Ensemencement de MRS contenant une concentration de lactose permettant d'obtenir une biomasse suffisante, par exemple 2-7% (p/p) ; incubation avec suivi de la DO à 580nm.
- La streptozotocine est préparée extemporanément, et est ajoutée en cours de fermentation, de façon à avoir une concentration finale qui est bactéricide pour la souche considérée. Cette concentration est souche-dépendante.
- Après incubation à 35-40°C, les cultures sont lavées deux fois avec du tryptone sel.
- Les culots sont repris dans du tryptone sel, puis des bouillons de MRSneutre sont remis en culture
- Incubation à 35-40°C, jusqu'à ce qu'il y ait croissance de la souche.
- Des isolements sont ensuite réalisés sur MRSneutre + Xgal + IPTG , puis incubés à 35-40°C sous CO2. Cette étape est un test colorimétrique permettant de déterminer si une souche bactérienne est lactose plus ou lactose moins : Si la souche est capable d'utiliser le lactose comme source de carbone, il y a production d'une substance bleue, la colonie sera donc bleue et dans le cas contraire (souche lactose moins), elle sera blanche.
- Les colonies blanches sont repiquées sur MRSneutre et incubées à 35-40°C.

L'invention sera davantage décrite à l'aide des exemples suivants, qui sont non-limitatifs.

**EXEMPLES DE REALISATION**

**EXEMPLE 1 : obtention de souches possédant un phénotype lactose moins**

Matériel et méthodes

Souches de départ (souches dites « mères »)

I-3431    déposée à la CNCM le 25/05/05
I-3434    déposée à la CNCM le 25/05/05
I-3435    déposée à la CNCM le 25/05/05

Test de sensibilité à la streptozotocine

Dans un premier temps, il est vérifié que les souches mères sont sensibles à l'antibiotique utilisé, la streptozotocine. La densité optique (DO) à 580nm est suivie sur des cultures à 42°C en bouillon MRS (Man Rogosa Sharp) neutre. Lorsque celle-ci atteint 0,1, la streptozotocine est ajoutée dans un tube afin d'avoir 50$\mu$g/ml au final (par exemple 100$\mu$l d'une solution à 5mg/ml pour 10ml de milieu).

Sélection de souches lactose moins

Les étapes suivantes sont réalisées afin d'obtenir des souches *Lactobacillus helveticus* lactose moins :

- 1er repiquage des souches *Lactobacillus helveticus* de départ (souches mères) à partir des cupules du stock de travail, sur bouillon MRS neutre, la nuit à 37°C ;
- 2ème repiquage à 1 % dans du MRS neutre.
- Incubation 16h à 37°C.
- Ensemencement de 10ml de MRS à 5% (p/p) de lactose, à 1 % et incubation à 40°C avec suivi de la DO à 580nm.
- La streptozotocine est préparée extemporanément, et est ajoutée au bout de 2h15 ou de 4h de fermentation, de façon à avoir une concentration finale de 50$\mu$g/ml.
- Après les 7h30 d'incubation à 40°C, les cultures sont lavées deux fois avec du tryptone sel.
- Les culots sont repris dans 2 ml de tryptone sel, puis des bouillons de MRSneutre sont ensemencés à 10%.
- Incubation à 37°C, jusqu'à ce qu'il y ait croissance de la souche.
- Des isolements sont ensuite réalisés sur MRSneutre + Xgal + IPTG , puis incubés à 37°C sous $CO_2$. Cette étape est un test colorimétrique permettant de déterminer si une souche bactérienne est lactose plus ou lactose moins : Si la souche est capable d'utiliser le lactose comme source de carbone, il y a production d'une substance bleue, la colonie sera donc bleue et dans le cas contraire (souche lactose moins), elle sera blanche.
- Les colonies blanches sont repiquées sur MRSneutre et incubées à 37°C.

Résultats

Cela a permis d'obtenir les souches suivantes, possédant un phénotype lactose moins qui ont ensuite fait l'objet de dépôts auprès de la CNCM (Collection Nationale de Cultures de Micro-organismes), 28 rue du Docteur Roux, 75724 Paris, France, en accord avec les dispositions du Traité de Budapest :

**I-3504**    déposée à la CNCM le 14/10/05 ; issue de la souche mère I-3431
**I-3505**    déposée à la CNCM le 14/10/05 ; issue de la souche mère I-3434
**I-3508**    déposée à la CNCM le 14/10/05 ; issue de la souche mère I-3435

Le phénotype lactose moins des souches obtenues est de nouveau confirmé sur un milieu gélose MRSneutre + Xgal + IPTG (test de coloration dit « blanc/bleu »)

**EXEMPLE 2 : Souches possédant un phénotype Lactose moins**

La souche **I-3504** , déposée le 14/10/05 auprès de la CNCM (Collection Nationale de Cultures de Micro-organismes), 28 rue du Docteur Roux, 75724 Paris, France, en accord avec les dispositions du Traité de Budapest, est une souche de *Lactobacillus helveticus* possédant les propriétés suivantes :

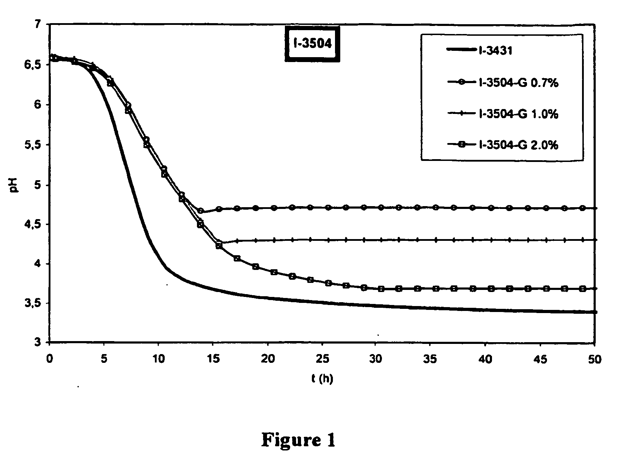

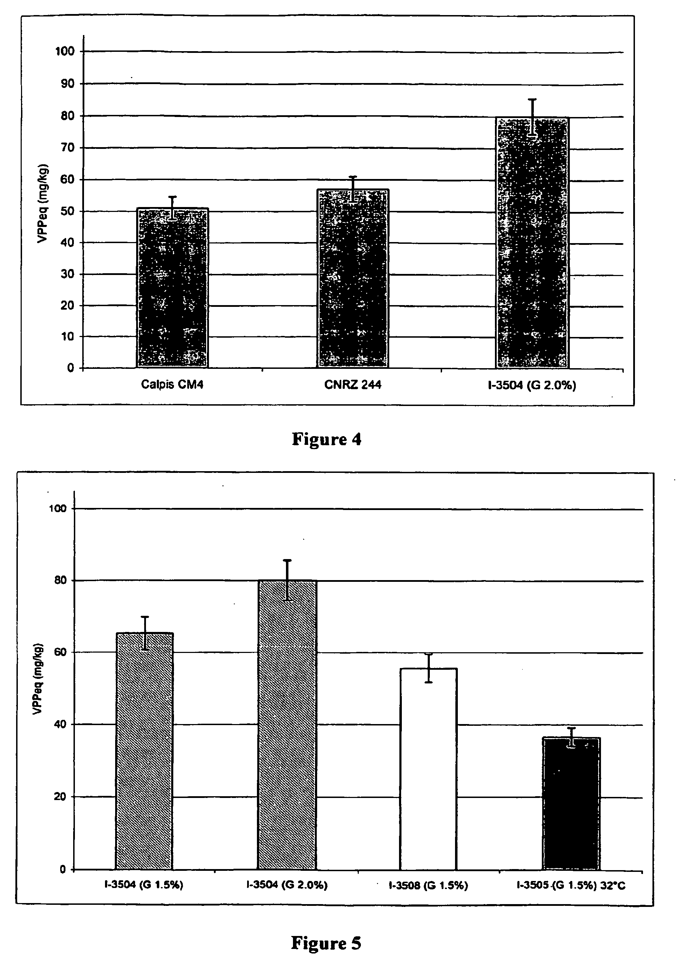

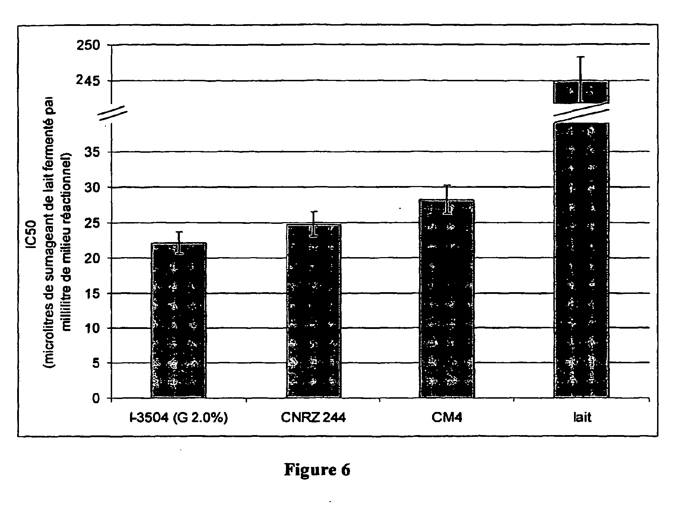

- Lactose moins ; Fructose moins
- production de IPP et VPP: voir Figures 4 et 5
- propriétés d'acidification: voir Figure 1
- inhibition de l'ACE : voir Figure 6

La souche **I-3505** déposée le 14/10/05 auprès de la CNCM (Collection Nationale de Cultures de Micro-organismes), 28 rue du Docteur Roux, 75724 Paris, France, en accord avec les dispositions du Traité de Budapest, est une souche de *Lactobacillus helveticus* possédant les propriétés suivantes :

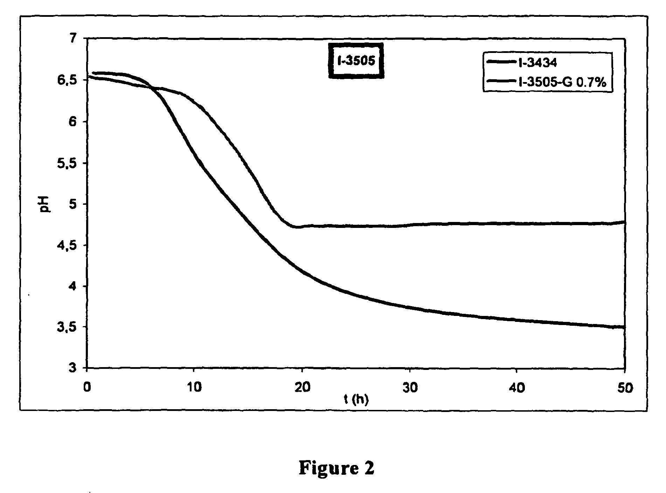

- Lactose moins ; Fructose moins
- production de IPP et VPP : voir Figure 5
- propriétés d'acidification: voir Figure 2

La souche **I-3508,** déposée le 14/10/05 auprès de la CNCM (Collection Nationale de Cultures de Micro-organismes), 28 rue du Docteur Roux, 75724 Paris, France, en accord avec les dispositions du Traité de Budapest, est une souche de *Lactobacillus helveticus* possédant les propriétés suivantes :

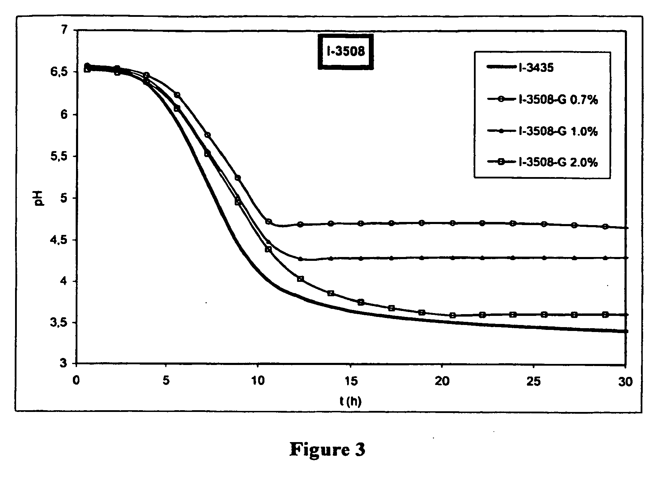

- Lactose moins ; Fructose moins
- production de IPP et VPP : voir Figure 5
- propriétés d'acidification : voir Figure 3

**1. Mesure des propriétés d'acidification des différentes souches variantes**

Les propriétés d'acidification sont évaluées comme suit :

Un milieu contenant 120g de poudre de lait écrémé (PLE) dans 930ml d'eau est pasteurisé 10min à 95°C. Ce milieu est ensemencé à 0.5%, puis soumis à fermentation à 37°C en présence de différentes concentrations de glucose. (L'abréviation Gx indique une concentration de x % (p/p) de glucose dans le milieu avant fermentation). Le pH est alors suivi en fonction du temps, pour différentes souches. Un prélèvement de milieu fermenté effectué à 30h de fermentation sera utilisé pour les mesures de production des tripeptides IPP et VPP ainsi que pour les mesures d'activité ACEI dont les résultats seront donnés ci-dessous.
Pour I-3504, on présente aussi les résultats de la souche mère (I-3431, souche lactose plus). (voir figure 1)
Pour I-3505, on présente aussi les résultats de la souche mère (I-3434, souche lactose plus). (voir figure 2)
Pour I-3508, on présente aussi les résultats de la souche mère (I-3435, souche lactose plus). (voir figure 3)
Les résultats présentés sur les **figures 1-3** montrent que les souches selon l'invention permettent avantageusement de finement contrôler le pH en fin de fermentation selon la quantité de glucose initialement présente dans le milieu. Par ailleurs, la fermentation par des souches lactose moins conduit avantageusement à des valeurs de pH élevées, qui donneront des produits alimentaires organoleptiquement acceptables.

**2. Mesure de la production des tripeptides IPP et VPP**

Matériel et méthode

Un échantillon de milieu laitier prélevé à 30 heures de fermentation, tel que décrit précédemment au point 1 est utilisé pour faire cette mesure.
L'analyse du contenu en peptides, notamment la teneur en tripeptides IPP et VPP, est conduite avec une méthode de chromatographie liquide CLHP couplée à un détecteur de type MS/MS comme décrit ci-après :
- Du fait des interférences inhérentes à l'analyse d'échantillons complexes, l'usage d'étalons internes deutérés ajoutés en quantité connue et maîtrisée au moment de la préparation de l'échantillon est fortement conseillée.
- La préparation de l'échantillon se fait par dilution du milieu fermenté dans un mélange d'eau, de méthanol et d'acide trifluoroacétique (50/50/0.1%), contenant 25 ppm d'étalon interne VPP deutéré (noté par la suite VPPd, de formule H-Val [D8]-Pro-Pro-OH, MM = 319,45 g/mol, disponible auprès de la société Bachem Chemicals, France) et 10 ppm d'étalon interne IPP deutéré (noté par la suite IPPd, de formule H-Ile [D10 N15]-Pro-Pro-OH, MM = 336,2 g/mol, disponible auprès de la société NEOMPS, Groupe SNPE, 7 rue de Boulogne, 67100 Strasbourg, France) dans un rapport de 1 à 3 (par exemple, pesée précise dans un eppendorf de 600 mg d'échantillon dans 1200 mg de mélange eau-méthanol-TFA contenant les étalons internes).
- Cet échantillon dilué est ensuite centrifugé à 14000 g pendant 15 minutes. Le surnageant clair obtenu, contenant les peptides générés pendant la fermentation, est ensuite dilué précisément au 1/50ème dans un mélange d'eau-méthanol (50/50, v/v) contenant 0,1% d'acide trifluoroacétique.

- La solution diluée ainsi obtenue est ensuite analysée dans un système chromatographique CLHP de type Agilent 1100 (société Agilent Technologies France, 1 rue Galvani 91745 Massy cedex, France), équipé d'une colonne adaptée à l'analyse des peptides, de type Waters Biosuite® (3 mm 2.1 x 150 mm, C18 PA-A, WAT186002427, Waters France, 5, Rue Jacques Monod, 78280 Guyancourt) à la température de 50°C, débit de 0.25 ml/min. Les peptides sont élués de façon classique avec un gradient croissant de solvant B (Acétonitrile + 0.100% d'acide formique) dans le solvant A (Eau + 0.106% acide formique), sur une durée de 35 min à 2 heures en fonction de la résolution chromatographique souhaitée. La méthode adaptée au dosage des peptides IPP et VPP utilise le gradient suivant :

| Temps | % Tampon A | % Tampon B |
|-------|------------|------------|
| 0 | 100 | 0 |
| 3 | 96 | 4 |
| 6 | 89 | 11 |
| 15 | 60 | 40 |
| 18 | 0 | 100 |
| 21 | 100 | 0 |
| 24 | 0 | 100 |
| 27,5 | 100 | 0 |
| 35 | 100 | 0 |

- La détection se fait à l'aide d'un détecteur spécifique de type MS/MS, par exemple avec un appareil à trappe ionique comme l'Esquire3000+ (Bruker Daltonique, rue de l'Industrie, 67166 Wissembourg Cedex), paramétré en ionisation electrospray en mode positif, soit pour l'analyse globale du contenu peptidique (mode MS-MS), soit pour la quantification précise et spécifique d'un peptide à partir de ses fragments caractéristiques (mode MRM). Dans le cas du dosage des peptides IPP et VPP, mais aussi des étalons internes IPPd et VPPd, ces peptides sont isolés à partir de leur masse spécifique (ions monochargés 312,2 Da pour VPP ; 326,2 Da pour IPP ; 320,2 Da pour VPPd ; 337,3 Da pour IPPd) et quantifiés à partir de l'intensité de leurs ions spécifiques après fragmentation (fragments >= 85 Da).

L'intégration des pics chromatographiques de chacun des peptides IPP, VPP et la comparaison aux surfaces de pic des étalons internes de concentrations connues IPPd et VPPd permet ensuite de calculer, par régression linéaire simple, la teneur initiale de l'échantillon en VPP et IPP (généralement exprimée en mg/kg ou ppm).

Résultats

| | VPPeq (mg/kg de milieu fermenté) |
|---|---|
| Calpis CM4 | 51 |
| CNRZ 244 | 57 |
| | |
| I-3504 (avec 2% (p/p) de glucose) | 80 |

La **figure 4** montre une comparaison de la production en tripeptides IPP et VPP de la souche **I-3504** (2% (p/p) de glucose sont ajoutés au milieu) par rapport à deux souches de l'art antérieur.

La souche CM4 de la société CALPIS est décrite dans le brevet EP 1 016 709 tandis que la souche CRNZ 244 est décrite dans la demande de brevet WO 2004/060073.

Il est clair sur cette figure que la souche **I-3504** (souche selon l'invention) a une production de tripeptides IPP et VPP bien supérieure à ces deux précédentes souches dans les mêmes conditions.

La **figure 5** montre une comparaison de la production en tripeptides IPP et VPP des souches selon l'invention entre elles.

### 3. Mesure de l'activité ACEI (Inhibition de l'Enzyme de Conversion de l'Angiotensine)

Matériel et méthode

Un échantillon de milieu laitier prélevé à 30 heures de fermentation, tel que décrit précédemment au point 1 est utilisé pour faire cette mesure.La présente méthode a pour base la méthode de Cushman et Cheng (Cushman et al. Biochem. Pharmacol. 1971. 20 :1637), adaptée pour la rendre compatible avec des échantillons de type produits laitiers fermentés.

**1. Préparation des réactifs et solutions**

**1.1 Tampon borate de sodium 0.1M pH8.3, additionné de 0.3M de NaCl**

Peser 6.1843g d'$H_3BO_3$ (Carlo Erba ref :402 766). Dissoudre dans environ 800 ml d'eau déminéralisée, ajuster à pH 8,3 avec une solution de NaOH puis ajouter 12,0 g de NaCl (concentration finale 0,3 M) et compléter à 1 litre avec l'eau déminéralisée.

**1.2 Préparation du substrat HHL : solution de Hip-His-Leu à 5mM dans tampon borate de sodium 0.1M pH8.3 avec 0.3M de NaCl**

Peser exactement 42.95 mg de peptide HHL anhydre (N-Hippuryl-Histidyl-Leucine tetrahydrate PM:501.5g, Sigma-Aldrich ref:53285-250 mg) et dissoudre dans environ 15 mL de tampon borate de sodium 0.1M pH8.3 avec 0.3M de NaCl puis compléter à 20mL avec ce même tampon.

**1.3 Préparation de la solution d'ACE à 0.1U/mL**

L'enzyme de conversion de l'angiotensine sous forme de poudre lyophilisée (origine : poumons de lapin, Sigma-Aldrich ref A6778, 0.25 unités) est solubilisée dans 2.5mL de tampon borate de sodium 0,1M pH 8,3 pour obtenir une solution à 0,1 U/mL. La solution ainsi préparée doit être utilisée, stockée à 4°C, au maximum 2 semaines pour préserver une activité enzymatique suffisante.

**2. Préparation des échantillons de lait fermenté**

Il est nécessaire de conditionner l'échantillon à un pH compris entre 8.0 et 8.5 de façon à être proche du pH optimal de l'ACE. Le lait fermenté est tout d'abord centrifugé, le pH du surnageant est ensuite ajusté entre 8.0 et 8.5 (idéalement pH 8.3) puis ultrafiltré à l'aide d'unité de filtration Vivaspin avec un seuil de coupure de 10 000 Daltons (Vivascience, France) afin d'éliminer les éléments interférents (protéines entières, sels de calcium).
Le protocole complet est le suivant :

- Déposer environ 6mL de lait fermenté dans un tube falcon 15mL après avoir préalablement pesé le tube vide. Peser la quantité de lait fermenté déposée dans le tube.

- Centrifuger à 14 000g pendant 10min à 10°C.

- Récupérer le surnageant

- Quantifier la proportion culot/surnageant ce qui permet si nécessaire de déterminer l'IC50 équivalente au produit d'origine et non à son seul surnageant.

- Prélever 2.0mL de surnageant dans un tube à essais et mesurer le pH.

- Ajouter le volume nécessaire de NaOH 2M afin d'obtenir un pH compris entre 8.0 et 8.5 (cible : 8.3) après ajout du tampon borate, puis agiter

- Ajouter le volume nécessaire de tampon borate pour diluer le surnageant de départ au ½ en tenant compte du volume exact de NaOH ajouté (par exemple : prise d'essai de surnageant = 2mL + 250µL NaOH + 1.75mL tampon borate)

- Vérifier que le pH final est compris entre 8.0 et 8.5. Au-delà, recommencer en ajoutant plus ou moins de NaOH. Un précipité se forme : il est dû aux sels de calcium.

- Ultracentrifuger l'échantillon à 12 000 g pendant 15 min à 10°C à l'aide d'unités de filtration Vivaspin 10 000 Daltons (capacité 4-6mL) afin d'obtenir un échantillon limpide.

**3. Mesure de l'inhibition de l'ACE par les échantillons de laits fermentés**

Lors de chaque série analyse, des témoins contrôles (0 et 100% activité ACE) doivent être préparés. Pour chaque échantillon, deux essais indépendants et un blanc échantillon sont réalisés pour chaque dilution. Il faut en effet ajuster au plus près (en diluant) la quantité d'échantillon nécessaire pour diminuer de 50% l'activité de l'ACE.
Pour cela :

- Déposer 80μL de l'échantillon conditionné à pH8.3 dans le tube blanc et dans les tubes essais
- Déposer 80μL d'eau déminéralisée dans les tubes contrôle 0 et 100%
- Ajouter 200μl de la solution de substrat HHL à 5mM dans tous les tubes. Agiter.
- Placer les tubes dans un bain d'eau thermostaté à 37°C, laisser s'équilibrer en température
- Déclencher la réaction enzymatique par ajout dans chaque tube de 20μL de la solution d'ACE à O.IU/mL, sauf pour les blanc échantillons et contrôle 0% pour lesquels il faut déposer 20μL de tampon borate pH 8.3.
- Laisser hydrolyser pendant 1h exactement à 37°C.
- Arrêter la réaction en ajoutant dans chaque tube 250μL de la solution d'HCl 1M.

Tableau récapitulatif de la composition des différents milieux réactionnels :

|  | Prise d'essai | Ajout du Substrat | Déclenchement hydrolyse |
|---|---|---|---|
| **100% contrôle** | 80μL eau déminéralisée | 200μL HHL | 20μL ACE |
| **0% contrôle** | 80μL eau déminéralisée | 200μL HHL | 20μL tampon borate |
| **Echantillon (essai)** | 80μL échantillon | 200μL HHL | 20μL ACE |
| **Blanc échantillon** | 80μL échantillon | 200μL HHL | 20μL tampon borate |

La lecture se fait par extraction du substrat hydrolysé (acide hippurique) et sa quantification à l'aide d'un spectrophotomètre, avec le protocole suivant :

Ajouter dans chaque tube 1.7mL acétate d'éthyle, agiter.
Centrifuger à 2000g pendant 5 min à 10°C
Prélever 1mL exactement du surnageant dans un micro-tube eppendorf
Evaporer l'acétate d'éthyle à 120°C pendant 10min dans un bloc chauffant
Ajouter exactement 1mL d'eau déminéralisée puis agiter 10 sec pour reprendre l'acide hippurique.
Lire l'absorbance à 228 nm dans des cuves adaptées à la lecture dans l'UV

Expression des résultats :

Le pourcentage d'inhibition de l'ACE est calculé comme suit:

$$\frac{(\text{Abs 100\% ctl} - \text{Abs 0\% ctl}) - (\text{Abs éch} - \text{Abs blanc éch})}{(\text{Abs 100\% ctl} - \text{Abs 0\% ctl})} \times 100$$

Avec Abs = absorbance à 228 nm après extraction de l'acide hippurique
ctl = contrôle
éch = échantillon
Pour un lait fermenté, nous exprimons l'IC50 comme étant la quantité de surnageant de ce lait fermenté qui inhibe 50% de l'activité enzymatique de l'ACE dans le milieu réactionnel, soit en microlitres de surnageant de lait fermenté par millilitre de milieu réactionnel.

Résultats

La **figure 6** montre une comparaison de l'activité inhibitrice de l'enzyme de conversion de l'angiotensine des différentes souches selon la présente invention comparées à d'autres souches de l'art antérieur.

En ordonnée est exprimée la concentration équivalente de lait fermenté par ml de milieu réactionnel nécessaire pour inhiber 50% de l'activité de l'enzyme de conversion de l'angiotensine (IC50). Plus cette concentration est élevée, moins la souche a donc de capacité à inhiber l'enzyme de conversion de l'angiotensine.

### EXEMPLE 3 : préparation d'un produit laitier (probiotique) selon l'invention

Un produit laitier fermenté est obtenu comme indiqué ci-dessous.

Du lait de grand mélange, préalablement écrémé, pré-pasteurisé et refroidi à 4°C est standardisé en protéines (4.0%) avec de la poudre de lait écrémé et additionné de glucose à raison de 1.8% (p/p). Le milieu laitier ainsi préparé est soumis à une pasteurisation (95°C-8 min). Après refroidissement à 37°C le milieu laitier est ensemencé avec une souche selon la présente invention à raison de $10^7$ UFC/mL et maintenu à 37°C pendant toute la durée de la fermentation. Lorsque le pH 3.8 est atteint, le caillé est soutiré de la cuve de fermentation pour subir un lissage (par passage au travers d'un filtre) et refroidi jusqu'à 10°C dans un échangeur à plaque. Le caillé lissé et refroidi est ensuite additionné d'une préparation de fruits (ayant un pH entre 4 et 4,1) à raison de 15% du produit fini, conditionné en flacons de 110 mL et stocké à 4°C pendant 28 jours.

Le produit ainsi préparé contient des tripeptides de séquence IPP/ VPP à raison de 65 mg/kg d'équivalent VPP. La teneur en peptides du produit est avantageusement stable durant toute la durée de vie du produit. La diminution de pH au cours du stockage réfrigéré est avantageusement négligeable (inférieure à 0,1 unité) et la population des souches selon la présente invention, reste avantageusement comprise entre $10^7$ et $10^8$ UFC/mL.

### Revendications

1. Souche de *Lactobacillus helveticus* n'ayant pas la capacité à transformer le lactose en acide lactique obtenue à partir des souches I-3431, 1-3434 et 1-3435 déposées à la CNCM le 25/05/05,
   et capable, par fermentation à une température entre 30 et 45°C, plus préférentiellement entre 32 et 43°C et encore plus préférentiellement à 37°C d'un milieu laitier contenant une quantité de glucose supérieure à 3% (p/p) et avec une teneur totale en protéines supérieure ou égale à 2% (p/p), préférentiellement entre 2 et 10% (p/p), plus préférentiellement entre 2,5 et 6% et encore plus préférentiellement égale à 4%,
   de produire les tripeptides de séquence IPP et/ou VPP en une quantité d'au moins 25 mg, préférentiellement au moins 50 mg, plus préférentiellement au moins 75 mg de VPPeq par kg de produit fermenté.

2. Souche de *Lactobacillus helveticus* selon la revendication 1, possédant au moins une mutation dans l'opéron lactose.

3. Souche de *Lactobacillus helveticus* selon l'une quelconque des revendications 1-2, telle qu'un milieu laitier à 4,0% de protéines totales contenant 1,0 % (p/p) de glucose fermenté par ladite souche de *Lactobacillus helveticus* pendant un temps maximal de fermentation de 30h, à une température entre 30 et 45°C, possède un pH supérieur ou égal à 4,0, préférentiellement supérieur ou égal à 4,1.

4. Souche de *Lactobacillus helveticus* selon l'une quelconque des revendications 1-3, qui possède en outre un phénotype fructose moins.

5. Souche de *Lactobacillus helveticus* selon la revendication 1, choisie parmi:

   I-3504    déposée à la CNCM le 14/10/05 ;
   I-3505    déposée à la CNCM le 14/10/05 ;
   I-3508    déposée à la CNCM le 14/10/05.

6. Utilisation d'une souche de *Lactobacillus helveticus* selon l'une quelconque des revendications 1-5, pour la préparation d'un produit alimentaire ou pharmaceutique, notamment d'un produit laitier fermenté.

**7.** Utilisation selon la revendication 6, telle que ledit produit alimentaire ou pharmaceutique a des propriétés anti-hypertensives.

**8.** Produit alimentaire, notamment produit laitier fermenté, comprenant au moins une souche de *Lactobacillus helveticus* selon l'une des revendications 1-5.

**9.** Produit alimentaire selon la revendication 8, contenant au moins $10^6$, préférentiellement au moins $10^7$, préférentiellement au moins $10^8$ UFC/mL de bactéries *Lactobacillus helveticus* vivantes.

**10.** Produit alimentaire selon l'une quelconque des revendications 8-9, contenant au moins 25 mg, préférentiellement au moins 50 mg, plus préférentiellement au moins 75 mg de VPPeq par kg dudit produit alimentaire.

**11.** Produit alimentaire selon l'une quelconque des revendications 8-10, comprenant en outre des préparations de fruit (s), notamment des morceaux et/ou du jus de fruit(s).

**12.** Produit alimentaire selon l'une quelconque des revendications 8-11, possédant un pH supérieur ou égal à 3,85, préférentiellement supérieur ou égal à 4,00, plus préférentiellement supérieur ou égal à 4,20.

**13.** Produit alimentaire selon l'une quelconque des revendications 8-12, qui possède des propriétés anti-hypertensives.

**14.** Procédé de préparation d'un produit alimentaire comprenant les étapes suivantes :

■ sélectionner une matière première contenant des protéines de lait, notamment des protéines contenant les séquences peptidiques IPP et/ou VPP;
■ sélectionner au moins une souche *Lactobacillus helveticus* selon l'une des revendications 1-5,
■ ensemencer ladite matière première avec ladite souche,
■ fermenter ladite matière première en présence de 1-3% (p/p) de glucose pendant 12-30 heures à 30-45°C, et en présence de ladite souche.

**15.** Procédé selon la revendication 14, dépourvu de toute étape de thermisation après fermentation.

**16.** Procédé d'obtention de souches *Lactobacillus helveticus* selon la revendication 1, comprenant les étapes suivantes :

■ fournir au moins une souche de *Lactobacillus helveticus* choisie parmi les souches I-3431, I-3434 et 1-3435 déposées à la CNCM le 25/05/05 ;
■ mettre en contact ladite au moins une souche avec une quantité efficace de streptozotocine en présence de lactose ;
■ isoler la ou les colonies de phénotype lactose moins selon la revendication 1.

**17.** Procédé selon la revendication 16, comprenant une étape de test colorimétrique sur un milieu contenant du Xgal et/ou du Sgal et/ou tout autre composé lié par une liaison β-galactosidique à du galactose et qui devient repérable suite à la rupture de cette liaison par ladite souche de *Lactobacillus helveticus.*

**Claims**

**1.** Strain of *Lactobacillus helveticus* not having the ability to convert lactose into lactic acid, obtained from strains I-3431, I-3434 and I-3435 deposited at the CNCM on 25/05/05,
and capable, by fermentation at a temperature between 30 and 45°C, more preferably between 32 and 43°C and still more preferably at 37°C of a dairy medium containing a quantity of glucose greater than 3% (w/w) and with a total proteins content greater than or equal to 2% (w/w), preferably between 2 and 10% (w/w), more preferably between 2.5 and 6% and still more preferably equal to 4%,
of producing the tripeptides of sequence IPP and/or VPP in a quantity of at least 25 mg, preferably at least 50 mg, more preferably at least 75 mg of VPPeq per kg of fermented product.

**2.** Strain of *Lactobacillus helveticus* according to claim 1, possessing at least one mutation in the lactose operon.

3. Strain of *Lactobacillus helveticus* according to any one of claims 1-2, such that a dairy medium with 4.0% total proteins containing 1.0 % (w/w) glucose fermented by said strain of *Lactobacillus helveticus* for a maximum fermentation time of 30 hours, at a temperature between 30 and 45°C, possesses a pH greater than or equal to 4.0, preferably greater than or equal to 4.1.

4. Strain of *Lactobacillus helveticus* according to any one of claims 1-3, which possesses moreover a fructose-negative phenotype.

5. Strain of *Lactobacillus helveticus* according to claim 1, chosen from:

> I-3504 deposited at the CNCM on 14/10/05;
> I-3505 deposited at the CNCM on 14/10/05;
> I-3508 deposited at the CNCM on 14/10/05.

6. Use of a strain of *Lactobacillus helveticus* according to any one of claims 1-5, for the preparation of a food or pharmaceutical product, in particular of a fermented dairy product.

7. Use according to claim 6, such that said food or pharmaceutical product has anti-hypertensive properties.

8. Food product, in particular fermented dairy product, comprising at least one strain of *Lactobacillus helveticus* according to one of claims 1-5.

9. Food product according to claim 8, containing at least $10^6$, preferably at least $10^7$, preferably at least $10^8$ CFU/mL of live *Lactobacillus helveticus* bacteria.

10. Food product according to any one of claims 8-9, containing at least 25 mg, preferably at least 50 mg, more preferably at least 75 mg of VPPeq per kg of said food product.

11. Food product according to any one of claims 8-10, also comprising preparations of fruit(s), in particular fruit pieces and/or juices.

12. Food product according to any one of claims 8-11, possessing a pH greater than or equal to 3.85, preferably greater than or equal to 4.00, more preferably greater than or equal to 4.20.

13. Food product according to any one of claims 8-12, which possesses anti-hypertensive properties.

14. Process for the preparation of a food product comprising the following steps:

> ■ selecting a raw material containing milk proteins, in particular proteins containing the peptide sequences IPP and/or VPP,
> ■ selecting at least one *Lactobacillus helveticus* strain according to one of claims 1-5,
> ■ seeding said raw material with said strain,
> ■ fermenting said raw material in the presence of 1-3% (w/w) glucose for 12-30 hours at 30-45°C, and in the presence of said strain.

15. Process according to claim 14, without any thermization stage after fermentation.

16. Process for obtaining *Lactobacillus helveticus* strains according to claim 1, comprising the following steps:

> ■ providing at least one strain of *Lactobacillus helveticus* chosen from strains I-3431, I-3434 and I-3435 deposited at the CNCM on 25/05/05;
> ■ bringing said at least one strain into contact with an effective quantity of streptozotocin in the presence of lactose;
> ■ isolating the colony or colonies of lactose-negative phenotype according to claim 1.

17. Process according to claim 16, comprising a colorimetric test stage on a medium containing Xgal and/or Sgal and/or any other compound which is linked by a β-galactoside bond to galactose and which can be tagged following the breaking of this bond by said strain of *Lactobacillus helveticus.*

**Patentansprüche**

1. Stamm von *Lactobacillus helveticus,* der Lactose nicht in Milchsäure umwandeln kann und der ausgehend von den Stämmen 1-3431, 1-3434 und 1-3435 erhalten wird, die bei der CNCM am 25/05/05 hinterlegt wurden, und der in der Lage ist, durch Fermentation eines Milchmediums, das eine Menge an Glucose von mehr als 3% (w/w) und einen Gesamtgehalt an Proteinen von mehr als oder gleich 2% (w/w), vorzugsweise zwischen 2 und 10% (w/w), stärker bevorzugt zwischen 2,5 und 6% und noch stärker bevorzugt gleich 4%, enthält, bei einer Temperatur zwischen 30°C und 45°C, stärker bevorzugt zwischen 32 und 43°C und noch stärker bevorzugt bei 37°C, Tripeptide mit der Sequenz IPP und/oder VPP in einer Menge von mindestens 25 mg, vorzugsweise mindestens 50 mg, stärker bevorzugt mindestens 75 mg, VPP-Äqu. pro kg fermentiertes Produkt zu produzieren.

2. Stamm von *Lactobacillus helveticus* nach Anspruch 1, der mindestens eine Mutation im Lactose-Operon besitzt.

3. Stamm von *Lactobacillus helveticus* nach einem der Ansprüche 1-2, derart, dass ein Milchmedium mit 4,0% Gesamt-Proteinen, das 1,0% (w/w) Glucose enthält und von dem Stamm von *Lactobacillus helveticus* während einer maximalen Fermentationsdauer von 30 Std. bei einer Temperatur zwischen 30 und 45°C fermentiert wird, einen pH von mehr als oder gleich 4,0, vorzugsweise mehr als oder gleich 4,1, aufweist.

4. Stamm von *Lactobacillus helveticus* nach einem der Ansprüche 1-3, der außerdem einen Fructose-minus-Phänotyp besitzt.

5. Stamm von *Lactobacillus helveticus* nach Anspruch 1, ausgewählt aus:

|  |  |
|---|---|
| I-3504 | hinterlegt bei der CNCM am 14/10/05, |
| I-3505 | hinterlegt bei der CNCM am 14/10/05, |
| I-3508 | hinterlegt bei der CNCM am 14/10/05. |

6. Verwendung eines Stamms von *Lactobacillus helveticus* nach einem der Ansprüche 1-5 zur Herstellung eines Nahrungsmittelprodukts oder pharmazeutischen Produkts, insbesondere eines fermentierten Milchprodukts.

7. Verwendung nach Anspruch 6, derart, dass das Nahrungsmittelprodukt oder pharmazeutische Produkt blutdrucksenkende Eigenschaften hat.

8. Nahrungsmittelprodukt, insbesondere fermentiertes Milchprodukt, das mindestens einen Stamm von *Lactobacillus helveticus* nach einem der Ansprüche 1-5 umfasst.

9. Nahrungsmittelprodukt nach Anspruch 8, das mindestens $10^6$, vorzugsweise mindestens $10^7$, bevorzugt mindestens $10^8$ CFU/ml an lebenden *Lactobacillus helveticus*-Bakterien enthält.

10. Nahrungsmittelprodukt nach einem der Ansprüche 8-9, das mindestens 25 mg, vorzugsweise mindestens 50 mg, stärker bevorzugt mindestens 75 mg, VPP-Äqu. pro kg des Nahrungsmittelprodukts enthält.

11. Nahrungsmittelprodukt nach einem der Ansprüche 8-10, das außerdem Zubereitungen von Frucht (Früchten), insbesondere Stücke und/oder Saft der Frucht (Früchte), umfasst.

12. Nahrungsmittelprodukt nach einem der Ansprüche 8-11, das einen pH von mehr als oder gleich 3,85, vorzugsweise mehr als oder gleich 4,00, stärker bevorzugt mehr als oder gleich 4,20, besitzt.

13. Nahrungsmittelprodukt nach einem der Ansprüche 8-12, das blutdrucksenkende Eigenschaften besitzt.

14. Verfahren zur Herstellung eines Nahrungsmittelprodukts, bei dem man in den folgenden Schritten:

■ ein Ausgangsmaterial wählt, das Milchproteine enthält, insbesondere Proteine, die die Peptidsequenzen IPP und/oder VPP enthalten,
■ mindestens einen *Lactobacillus helveticus*-Stamm nach einem der Ansprüche 1-5 auswählt,
■ das Ausgangsmaterial mit dem Stamm beimpft,

■ das Ausgangsmaterial in Gegenwart von 1-3% (w/w) Glucose für 12-30 Stunden bei 30-45°C und in Gegenwart des Stamms fermentiert.

15. Verfahren nach Anspruch 14, das keinerlei Thermisationsschritt nach der Fermentation umfasst.

16. Verfahren zum Erhalten von *Lactobacillus helveticus*-Stämmen nach Anspruch 1, bei dem man in den folgenden Schritten:

■ mindestens einen Stamm von *Lactobacillus helveticus* bereitstellt, der aus den bei der CNCM am 25/05/05 hinterlegten Stämmen 1-3431, 1-3434 und 1-3435 ausgewählt wird,
■ den mindestens einen Stamm mit einer wirksamen Menge an Streptozotozin in Gegenwart von Lactose in Kontakt bringt,
■ die Kolonie oder Kolonien mit Lactose-minus-Phänotyp nach Anspruch 1 isoliert.

17. Verfahren nach Anspruch 16, umfassend einen Schritt mit einem kolorimetrischen Test auf einem Medium, das XGal und/oder Sgal und/oder jede andere Verbindung enthält, die durch eine β-galactosidische Bindung an Galactose gebunden ist und die nach der Lösung dieser Bindung durch den Stamm von *Lactobacillus helveticus* nachweisbar wird.

**Figure 1**

**Figure 2**

**Figure 3**

**Figure 4**

**Figure 5**

**Figure 6**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0583074 A
- EP 1016709 A
- WO 2004060073 A

**Littérature non-brevet citée dans la description**

- **MASUDA O. et al.** *J. Nutr.,* 1996, vol. 126, 3063-8
- **YAMAMOTO N. et al.** *Biosci. Biotech. Biochem.,* 1994, vol. 58, 776-8
- **HATA et al.** *Am. J. Clin. Nutr.,* 1996, vol. 64, 767-71
- **MIZUNO et al.** *British Journal of Nutrition,* 2005, vol. 94 (1), 84-91
- **JAUHIAINEN et al.** *Am. J. of Hypertension,* 2005, vol. 18, 1600-1605
- **CUSHMAN et al.** *Biochem. Pharmacol.,* 1971, vol. 20, 1637